# EUROPEAN PATENT SPECIFICATION

(11) **EP 3 149 155 B1**
(45) Date of publication and mention of the grant of the patent: **23.09.2020**
(21) Application number: 15802806.8
(22) Date of filing: 02.06.2015
(51) Int. Cl.: A61K 38/18, A61K 35/30, A61K 31/519, A61K 31/4418, C12N 5/079, A61P 25/00

(54) **METHODS OF INDUCING MYELINATION AND MATURATION OF OLIGODENDROCYTES**
VERFAHREN ZUR INDUZIERUNG DER MYELINISIERUNG UND REIFUNG VON OLIGODENDROZYTEN
PROCÉDÉS D'INDUCTION DE LA MYÉLINISATION ET DE MATURATION DES OLIGODENDROCYTES

(30) Priority: 02.06.2014 US 201462006285 P
(43) Date of publication of application: 05.04.2017
(73) Proprietor: Kadimastem Ltd., 74140 Nes-Ziona (IL)
(72) Inventor: IZRAEL, Michal, 76620 Rechovot (IL); REVEL, Michel, 76302 Rechovot (IL); HASSON, Arik, 55401 Kiryat-Ono (IL); CHEBATH, Judith, 76286 Rechovot (IL)
(74) Representative: Straus, Alexander
(86) International application number: PCT/IL2015/050563
(87) International publication number: WO 2015/186128

(56) References cited:
- WO-A1-2012/096705
- WO-A2-2008/026198
- WO-A2-2012/058243
- WO-A2-2013/186777
- FADI J. NAJM ET AL: "Rapid and robust generation of functional oligodendrocyte progenitor cells from epiblast stem cells", NATURE METHODS, vol. 8, no. 11, 1 November 2011 (2011-11-01), pages 957-962, XP055082813, DOI: 10.1038/nmeth.1712
- SABO, JENNIFER K. ET AL.: 'Remyelination is altered by bone morphogenic protein signaling in demyelinated lesions.' THE JOURNAL OF NEUROSCIENCE, [Online] vol. 31.12, 23 March 2011, pages 4504 - 4510, XP055240870 Retrieved from the Internet: <URL:http://www.jneurosci.org/ content/31/12/4504.full>
- IZRAEL, MICHAL ET AL.: 'Human oligodendrocytes derived from embryonic stem cells: Effect of noggin on phenotypic differentiation in vitro and on myelination in vivo.' MOLECULAR AND CELLULAR NEUROSCIENCE vol. 34.3, 28 December 2006, pages 311 - 316, XP005907285

## Description

### FIELD AND BACKGROUND OF THE INVENTION

The present invention relates to ex vivo methods of inducing myelination in a patient suffering from demyelination. The present invention further relates to ex vivo methods of inducing commitment or maturation of oligodendrocyte cells.

Myelin, the fatty substance which wraps certain axons and nerve fibers, provides essential insulation, and enables the conductivity of nerve cells which transmit electrical messages to and from the brain.

Aberration in myelination can lead to pathologies of multiple origins in the central nervous system. These include for example, autoimmune diseases (e.g Multiple Sclerosis), congenital leukodystrophies (e.g Pelizaeus-Merzbacher, vanishing white matter, adrenoleukodystrophy), infectious diseases (e.g. progressive multifocal leukoencephalopathy, postinflammatory demyelinated lesions), neurodegenerative diseases (e.g. multisystem degeneration), vascular diseases (vascular leukoencephalopathies, subcortical infarcts), congenital genetic defects (e.g. amyotrophic lateral sclerosis [ALS], Alzheimer disease, Parkinson disease) and brain and spinal cord trauma or injuries which are demyelinative and possibly neoplasms (e.g. oligodendrio-glioma).

In Multiple Sclerosis (MS) and other inflammatory demyelinating diseases re-myelination by resident endogenous oligodendrocyte precursor cells (OPC) halts as disease progresses. This leads to increased lesion load, brain atrophy and functional decline. Pharmacological potentiation of endogenous OPC differentiation, maturation and subsequent re-myelination will serve as a promising therapeutic approach for MS.

In human, CNS myelination starts perinatally, is active in the two first decades of life, and continues at slow pace throughout life. During development, neural stem cells (Pax6, Nkx2.2, Nkx6.2 positive) are converted to oligodendrocyte progenitors cells (OPCs), expressing Olig1/2, Hes 5, as well as Sox10, and bearing surface markers as NG2 and PDGF receptor a, mediating the proliferating and survival effect of PDGFa. OPCs are bipolar or contain very few extensions and can migrate and proliferate. Progression to pre-myelinating stage involves a decrease in proliferation (together with a loss of the cell surface PDGF receptor a), extension of processes, appearance of the specific marker O4 and induction of transcription factors such as MRF. Once post mitotic oligodendrocytes have contacted axons, they wrap axons with myelin fibers in about 12 hours, in a complex process requiring activation of the oligodendrocyte FYN kinase and synthesis of several proteins among which is Myelin basic protein MBP. Early development of the oligodendrocyte lineage is positively regulated by Sonic Hedghog, FGF2, and antagonized by Bone morphogenetic protein (BMPs) pathways. During Myelination and re-myelination BMP signaling is also one of the major obstacles to oligodendrocyte function.

BMPs, which are members of the transforming growth factor b (TGF-b) family, bind to heteromeric complexes of BMP receptors type I (or Activin-like receptors Alks) and type II (e.g., BMPR-II) serine threonine kinase receptors and activate downstream gene expression, including inhibitors of DNA binding Id2 to Id4 mainly through BMP receptor activated Smads (Smad1/5/8). Functions of BMPs are regulated by secreted antagonistic regulators such as noggin, chordin, follistatin, neurogenesin-1, which bind BMPs and prevent their interaction with their receptors. During the pre-myelination and myelination stages a protein SIP1 interacting and inhibiting the function of SMAD 1/5/8 is expressed in oligodendrocytes and requested for expression of the transcription factors MyRF or MRF (Myelin Regulatory Factor).

Human embryonic stem cell (hESC)-derived oligodendrocytes or oligodendrocyte progenitors may be experimentally utilized to solve fundamental questions of oligodendrocyte development, or utilized for screening for drugs to treat diseases involving oligodendrocyte dysfunction or degeneration. Currently, the protocols utilized to derive oligodendrocytes from hESCs consist of significant variations in culture style and time-length. Glial progenitors derived from human ES cells express various members of the BMP family (Izrael et al, Mol Cell Neurosci. 34(3):310-23, 2007) as well as their receptors. These cells proliferate in the presence of bFGF and EGF, can be stored frozen, and start to differentiate when plated in a medium without growth factors. The inventors of the present invention have shown that O4 positive and MBP positive cells with multiple processes appear when the inhibitor of BMP signaling noggin was added to the cells in medium without growth factor (Izrael et al Mol Cell Neurosci. 2007). The WO 2013186777 A2 discloses a composition for treating a neuroinflammatory disease or nervous system damage comprising at least a blocking agent of Bone Morphogenic Protein signal transduction with a preferred embodiment relating to a demyelinating disease. Najm et al. (Najm, FJ et al., Nature Methods, 8(11), p. 957-62, September 25, 2011) describe the differentiation of oligodendrocyte progenitor cells from epiblast stem cells into myelinating oligodendrocytes. WO 2012096705 A1 discloses treating epiblast stem cells with SB431542 or dorsomorphin or LDN-193189 to differentiate tehm into neuroectodermal lineage and the use of these cells to treat demyelating diseases and use for drug testing. WO 2012058243 A2 describes the differentiation of oligodendrocytes and their use as diagnostic tool or pharmaceutical, while disclosing special surface markers of differentiated cells as well as success of transplantation of said cells into an animal demyelination model organism.

### SUMMARY OF THE INVENTION

According to one aspect of the present invention there is provided an ex vivo method of inducing commitment or maturation of oligodendrocyte cells, the oligodendrocyte cells having been ex-vivo differentiated from glial progenitor cells derived from pluripotent stem cells (PSC) in a medium comprising DMEM/F12, 1% B27, 0.5% N2, Vitamin C, wherein the method comprises contacting said glial progenitor cells in said medium with 4-(6-(4-(piperazin-1-yl)phenyl)pyrazolo[1,5-a]pyrimidin-3-yl)quinolone (LDN-193189).

According to some embodiments, said demyelination is from a disease selected from the group consisting of multiple sclerosis, stroke, inflammation, and spinal cord injury.

According to yet another aspect of the present invention there is provided an isolated committed or mature oligodendrocyte cell generated according the above method. Said oligodendrocyte cell expresses a marker selected from the group consisting of PDGFRA, olig2, O4, O1, myelin-associated glycoprotein (MAG), PLP, MBP, myelin oligodendrocyte glycoprotein (MOG).

According to an additional aspect of the present invention there are provided the committed or mature oligodendrocyte cells, generated according to any of the above methods, for use in the treatment of a medical condition of the CNS.

According to yet an additional aspect of the present invention there is provided, a pharmaceutical composition comprising as an active ingredient the oligodendrocyte cells, generated according to any of the above methods, and a pharmaceutically acceptable carrier or diluent.

The oligodendrocyte cell may be a human committed or mature oligodendrocyte cell.

According to still further features in the described preferred embodiments said mature oligodendrocyte cell further expresses a marker selected from the group consisting of olig2, olig1, sulfatide marker, galactocerebrosides (O1, GalC), Nkx2.2, Sox10, oligodendrocyte specific protein (OSP/claudin-11), , 2',3'-cyclic nucleotide-3'-phosphodiesterase (CNPase), glutathione-S-transferase (GST), adenomatous polyposis coli (APC); MOSP and Oligodendrocyte NS-1.

According to still further features in the described preferred embodiments the committed or mature oligodendrocyte cell has morphology of multipolar shape.

According to still further features in the described preferred embodiments the committed or mature oligodendrocyte phenotype comprises in vivo and in vitro myelin production.

The committed or mature oligodendrocyte phenotype may comprise a mature oligodendrocyte marker expression.

According to further features in the described preferred embodiments the mature oligodendrocyte marker is selected from a group consisting of O4, O1, PLP, MBP, MAG and MOG.

According to still further features in the described preferred embodiments the mature oligodendrocyte phenotype comprises a mature oligodendrocyte structural phenotype.

According to still further features in the described preferred embodiments the mature oligodendrocyte structural phenotype is branched and ramified.

According to still further features in the described preferred embodiments the medical condition is associated with insufficient myelination

According to still further features in the described preferred embodiments the medical condition is selected from the group consisting of multiple sclerosis, stroke, inflammation and spinal cord injury and an autoimmune disease.

According to still a further aspect of the present invention there is provided a method of determining an effect of a drug on differentiation of human oligodendrocyte progenitor cell toward differentiated and mature oligodendrocyte, the method comprising:
(a) subjecting committed OPC or mature oligodendrocyte generated according to any of the above methods to the drug; and
(b) determining at least one of a structural or functional phenotype of the treated cell as compared to an untreated cell, thereby determining an effect of the drug on the cell functionality.

The present invention successfully addresses the shortcomings of the presently known configurations by providing novel methods of inducing maturation of oligodendrocytes and methods of using committed oligodendrocyte progenitor cells or mature oligodendrocytes for the treatment of medical conditions of the CNS.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

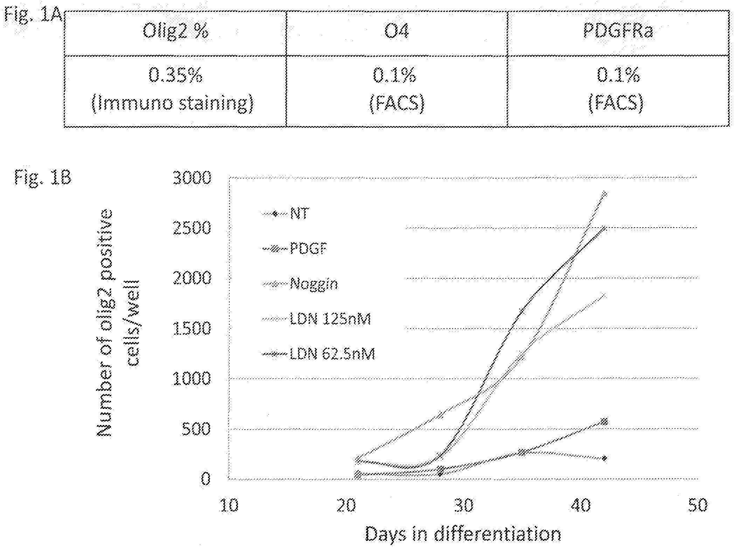

FIGs. 1A-B demonstrate that Noggin and LDN-193189 stimulate the conversion of neural stem cells to Olig2 positive cells with the same kinetics. Figure 1A shows that Neural Stem Cells (NSCs) at passage 5 prior to the addition of differentiation medium contain very low percentage of cells of the oligodendrocyte lineage (olig2 positive or PDGFRA positive). Figure 1B demonstrates the comparison between the number of olig2 positive cells in cultures non-treated or treated with PDGF, Noggin or LDN-193189. Time points used for analysis are 21, 28, 35 and 42 days of differentiation, for each time point 20 fields were analyzed.

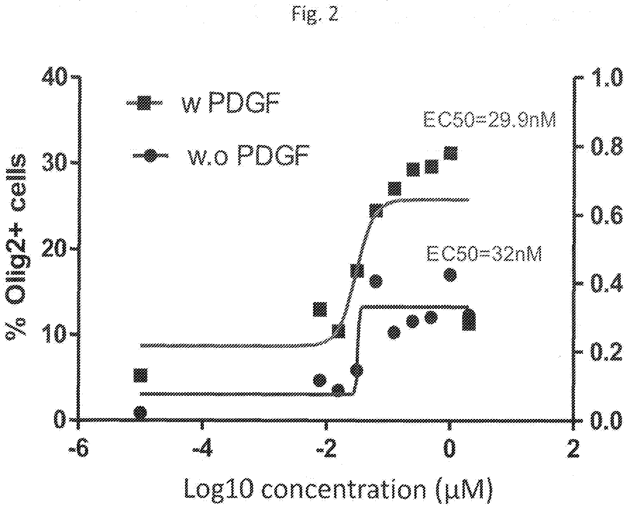

FIG. 2 depicts LDN-193189 dose-response curves which show a plateau of activity at 62.5 to 125 nM and EC50 at about 30nM. Cells at passage five were cultured in 96 well plates in GM for four days, then treated with LDN-193189 from 2µM to 0.0075uM in differentiation medium without noggin, with or without PDGF (20ng/ml) for 21 days.

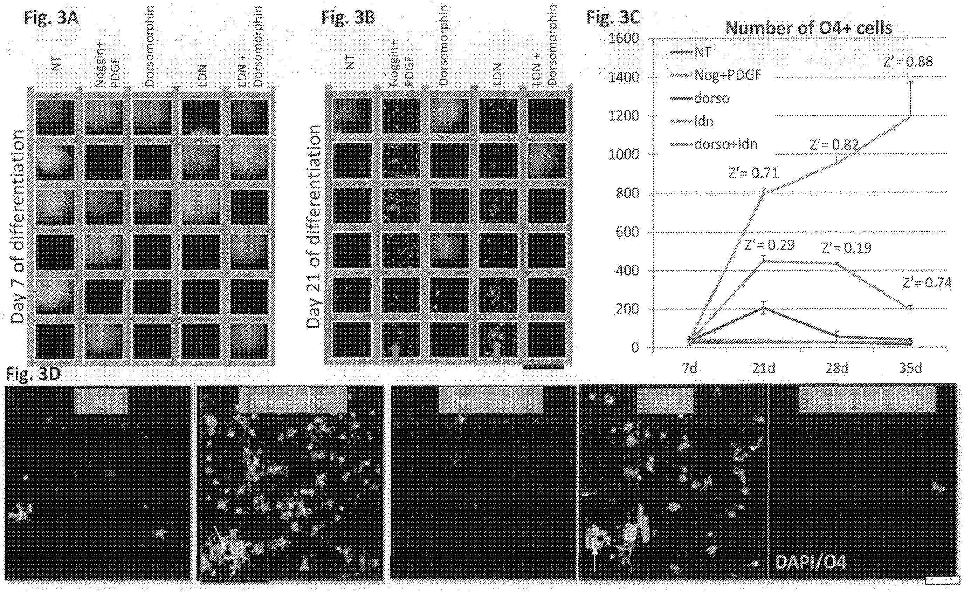

FIGs. 3A-D demonstrate the effect of Noggin and PDGF; Dorsomorphin; LDN-193189 and Dorsomorphin, on human OPC differentiation. Figure 3A shows that no O4+ cells were observed on day seven of differentiation. Each column represent a treatment, for each treatment 6 well replicas were conducted, 36 fields were analyzed for each well. Figure 3B demonstrates an increase in the number of O4+ cells (green) on day 21 of differentiation in Noggin&PDGF and LDN-193189 treatments (column 2 and 4 respectively), whereas Dorsomorphin and LDN+Dorsomorphin abolished hOPC differentiation (Column 3 and 5 respectively). NT=none treated media, Noggin+PDGF= media supplemented with 50ng/ml Noggin and 20 ng/ml PDGF, Dorsomorphin= Media supplemented with 2µM Dorsomorphin, LDN=193189 media supplemented with 125nM LDN-193189 and Dorsomorphin+LDN-193189 media supplemented with 2µM Dorsomorphin and 125nM LDN-193189. Figure 3C demonstrates the kinetics (day 7, 21, 28 and 35) of hOPC differentiation as was measured by of O4+ cells count. From day 21 Z' factor was calculated (presented on graph). Noggin&PDGF and LDN presented valid Z'factor (Z'>0.25) in most of tested time points indicating a significant difference (P<0.0001). Figure 3D shows O4 staining (green) and DAPI (blue). Enlargement (inset) of representative O4+ cell with myelin sheaths (arrow) is presented in Noggin+PDGF and LDN-193189 treatment images. Representative figure, for each field is presented, Scale bar for A= 660µm and for D= 100µm.

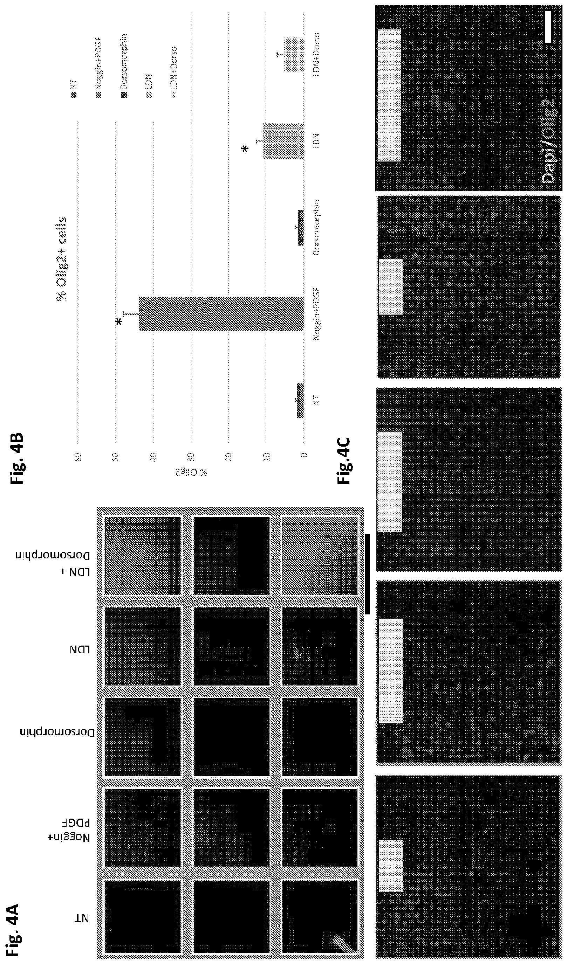

FIGs. 4A-C demonstrate the effect of Noggin and PDGF; Dorsomorphin; LDN-193189 and Dorsomorphin, on human hOPC early differentiation and commitment. Figure 4A shows Olig2 staining (red) on day 21 of hOPC differentiation. Noggin+PDGF and LDN-193189 treatments increased the number of Olig2+ (column 2 and 4 respectively) whereas LDN+Dorsomorphin decreased hOPC commitment (Column 5). NT=none treated media, Noggin+PDGF= media supplemented with 50ng/ml Noggin and 20 ng/ml PDGF, Dorsomorphin= Media supplemented with 2µM Dorsomorphin, LDN= media supplemented with 125nM LDN-193189 and Dorsomorphin+LDN media supplemented with 2µM Dorsomorphin and 125nM LDN-193189. Each column represent a treatment with 6 well replicas, 36 fields were analyzed for each well. Figure 4B demonstrates that the percentage of Olig2+, Noggin+PDGF and LDN-193189 significantly increased the number of Olig2+ cells (P<0.01), whereas LDN+Dorsomorphin decreased hOPC commitment when compared to LDN-193189. Figure 4C shows representative field for each treatment, Dapi (blue) and Olig2+ (red). Scale bar for A= 660µm and for C= 100µm.

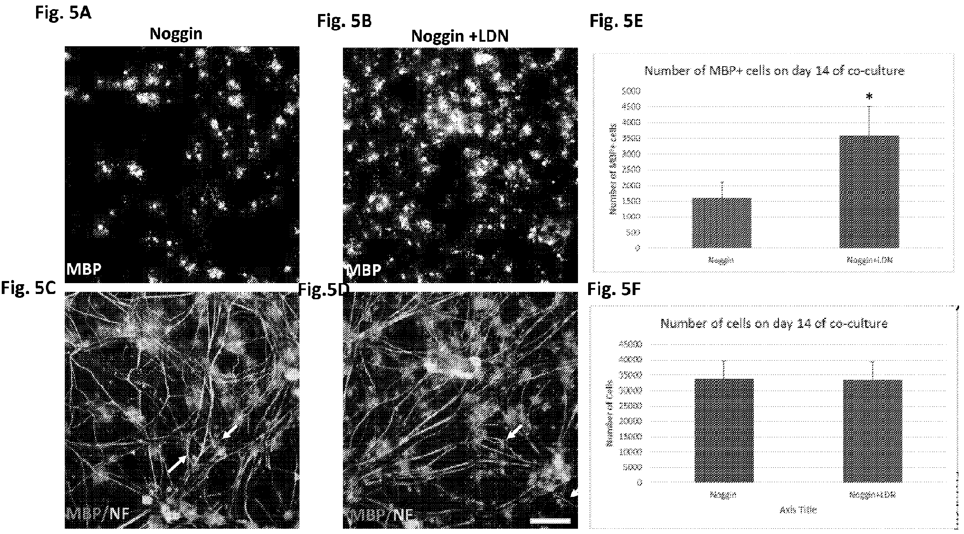

FIGs. 5A-F demonstrates that LDN-193189 increased the number of MBP+ cells. Figures 5A-D : 14 days co-culture between human oligodendrocytes and DRGs neuron axons treated with Noggin (5A+5C) and Noggin+LDN (5B+5D), MBP staining (5A+5B, white color) and an overlay images between MBP staining (red), Neurofilament-160 staining (green) and Dapi (blue), Myelin fibers are observed in both treatments (arrows). Noggin treatment=MYL media supplemented with 50ng/ml Noggin. Noggin+ LDN-193189 treatment=MYL media supplemented with 50ng/ml Noggin and 125nM LDN-193189. Figure 5E shows that a significant increase (P<0.05) in the number of mature oligodendrocytes (MBP+ cells) was observed when LDN-193189 was added (red bar) compared to Noggin treatment alone (blue). Figure 5F shows that no difference was observed in the number of total cell count between the treatments. Scale bar for 5A-D =100µm.

### DESCRIPTION OF THE SPECIFIC EMBODIMENTS

The present invention discloses methods of inducing myelination in a patient suffering from demyelination. The present invention further discloses methods of inducing commitment of glial restricted cells toward oligodenrocytes lineage and induce commitment and maturation of oligodendrocyte cells.

The present invention disclosed for the first time, that a small molecule LDN- 193189, an inhibitor of the activity of the BMPR of type I Alk2 and Alk3, stimulates the differentiation of glial progenitors towards the oligodendrocytic lineage. LDN- 193189 stimulates the formation of olig2 positive cells from olig2 negative precursors and the formation of O4 positive cells from the olig2 positive progenitors. The EC50 of LDN activity on olig2 progenitors is similar to the EC50 of Alk 3 inhibition. On the contrary Dorsomorphin, a molecule from which LDN-193189 was derived by chemical modification and which inhibit the same Alks, strongly inhibits the differentiation of olig2 cells or the conversion of olig2 positive cells into O4 positive cells, even at concentrations of the nmolar range. Dorsomorphin affects the activity of other kinases, suggesting other kinases are important for oligodendrocyte development.

The principles and operation of the present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

The main function of oligodendrocytes is the myelination of nerve cells. The myelin sheath serves as insulation, resulting in decreased ion leakage, lower capacitance of the cell membrane and as a result allows salutatory nerve conduction (i.e. the fast propagation of neuroelectrical impulses). The myelin sheaths and several of its protein components play essential roles in protection of neurons against disintegration and death. Diseases that result in injury of oligodendroglial cells include demyelinating diseases such as multiple sclerosis, infectious or ischemic demyelinating diseases and various types of inherited or acquired leukodystrophies. Information about functions of myelin and diseases caused by alterations of myelin can be found in Lazzarini et al, eds (2004) Myelin biology and disorders, Elsevier Academic Press, San Diego, CA).

All the described attempts to produce fully differentiated and mature oligodendrocytes from human ES cells convey that procedures which have been successful with murine ES cells cannot be extrapolated to human ES cells. Thus, the need for ex vivo or in vivo production of fully differentiated and mature oligodendrocytes propagated and expanded from human ES cells is still unmet.

As is described herein below and in the Examples section which follows, the present inventors have uncovered, through laborious experimentation, a novel procedure for inducing maturation of oligodendrocyte cells. The general guidelines of the methods of the present invention are further provided in the Examples section which follows.

The phrase "glial cells", also termed interchangeably herein as "glia", are non-neuronal cells that provide support and nutrition, maintain homeostasis, form myelin, and participate in signal transmission in the nervous system. Examples of glial cells as disclosed here may include but are not limited to astrocytes and oligodendrocytes (mature and precursor, as further described herein below).

As used herein the term "oligodendrocyte" refers to both oligodendrocyte precursor cells (OPCs) and mature well-differentiated oligodendrocytes. The function of these cells is described above. Mature oligodendrocytes may be distinguished from OPCs both by structural and functional phenotypes.

Examples of a mature oligodendrocyte functional phenotype include, but are not limited to one or more, marker expression such as proteolipid protein (PLP), MBP expression, myelin-associated glycoprotein (MAG), myelin oligodendrocyte glycoprotein (MOG), sulfatide marker (O4), in addition to galactocerebrosides (O1, GalC).

Examples of mature oligodendrocyte structural phenotype include, but are not limited to, a branched and ramified phenotype and formation of myelin membranes.

Examples of an OPC functional phenotype include, but are not limited to, mitotic (i.e. that can divide and be expanded for three or more passages in culture) and migratory capacities as well as the potential to differentiate into a myelinating phenotype to effect myelination in vivo and in vitro.

Examples of OPC marker expression include, but are not limited to, PDGF-receptor, O4 sulfatide marker, Nkx2.2, Olig1/2, oligodendrocyte specific protein (OSP), 2',3'-cyclic nucleotide-3'-phosphodiesterase (CNP), adenomatous polyposis coli (APC), NG2 (Chondroitin sulfate proteoglycan), A2B5, GD3 (ganglioside), nestin, vimentin and E- or PSA-NCAM

Examples of OPC structural phenotype include, but are not limited to elongated, bipolar or multipolar morphology. For example only OPCs, but not mature oligodendrocytes and astrocytes, incorporate bromodeoxyuridine (BUdR), a hallmark of mitosis.

As used herein the phrase "neuronal cells" refers to the polar cells of the vertebrate nerve system which are specialized for the transmission of nerve impulses. Such cells typically display neuronal cell structure and express at least one neuronal marker. Examples of such markers include, but are not limited to Peripherin, Choline Acetyltransferase [ChAT], Chromogranin A, DARPP-32, GAD65, GAD67, GAP43, HuC, HuD, Alpha internexin, MAP5, MAP-2 A&B, Nestin, NeuN, Neurofilament L,M, H, Neuron-Specific Enolase (gamma-NSE), P75, low affinity NGF receptor, Peripherin, PH8, Protein Gene Product 9.5 (PGP9.5), Serotonin Transporter (SERT), Synapsin, Tau, Thy-1, TrkA, TrkB, Tryptophan Hydroxylase (TRH) Beta III Tubulin, TUC-4 (TOAD/Ulip/CRMP) Tyrosine hydroxylase (TH) Vanilloid Receptor Like Protein 1 (VRL-1), Vesicular GABA Transporter (VGAT) Vesicular Glutamate Transporter 1 (VGLUT1; BNPI) and VGLUT2 (all available at Chemicon/Millipore, Temecula, CA).

Oligodendrocytes, neurons and other glial cells of this aspect of the present invention are generated by growing human stem cells under conditions which induce the differentiation of the human stem cells into the neuronal and glial cells. These conditions comprise the presence of retinoic acid and an agent capable of down-regulating Bone Morphogenic Protein (BMP) activity.

As used herein the phrase "stem cells" " refers to cells which are capable of differentiating into other cell types (i.e., neuronal or glial cells as described herein) having a particular, specialized function (*i.e*., "fully differentiated" cells) or self-renew while remaining in an undifferentiated state. Examples of stem cells which can be used in accordance with this aspect of the present invention include, but are not limited to, embryonic stem cells, induced pluripotent stem cells as well as fetal or adult stem cells (e.g., mesenchymal). According to currently known preferred embodiment of the present invention, the stem cells are pluripotent stem cells (embryonic stem cells and induced pluripotent stem cells).

It will be appreciated that undifferentiated stem cells are of a distinct morphology, which is clearly distinguishable from differentiated cells of embryo or adult origin by the skilled in the art. Typically, undifferentiated stem cells have high nuclear/cytoplasmic ratios, prominent nucleoli and compact colony formation with poorly discernable cell junctions. Additional features of undifferentiated stem cells are further described hereinunder.

The stem cells can be obtained using well-known cell-culture methods.

For example, human embryonic stem cells can be isolated from human blastocysts or delayed blastocyst stage (as described in WO2006/040763). Human blastocysts are typically obtained from human *in vivo* preimplantation embryos or from *in vitro* fertilized (IVF) embryos. Alternatively, a single cell human embryo can be expanded to the blastocyst stage. For the isolation of human ES cells the zona pellucida is removed from the blastocyst and the inner cell mass (ICM) is isolated by immunosurgery, in which the trophectoderm cells are lysed and removed from the intact ICM by gentle pipetting. The ICM is then plated in a tissue culture flask containing the appropriate medium which enables its outgrowth. Following 9 to 15 days, the ICM derived outgrowth is dissociated into clumps either by a mechanical dissociation or by an enzymatic degradation and the cells are then re-plated on a fresh tissue culture medium. Colonies demonstrating undifferentiated morphology are individually selected by micropipette, mechanically dissociated into clumps, and re-plated. Resulting ES cells are then routinely split every 1-2 weeks. For further details on methods of preparation human ES cells see Thomson et al., [U.S. Pat. No. 5,843,780; Science 282: 1145, 1998; Curr. Top. Dev. Biol. 38: 133, 1998; Proc. Natl. Acad. Sci. USA 92: 7844, 1995]; Bongso et al., [Hum Reprod 4: 706, 1989]; Gardner et al., [Fertil. Steril. 69: 84, 1998].

It will be appreciated that commercially available stem cells can be also be used with this aspect of the present invention. Human ES cells can be purchased from the NIH human embryonic stem cells registry (<http://escr.nih.gov>). Non-limiting examples of commercially available embryonic stem cell lines are BG01, BG02, BG03, SA01, TE03 (13), TE04, TE06 (16), HES-1, HES-2, HES-3, UC01, UC06, WA01, WA07 and WA09 (see also Example 1 of the Examples section which follows).

Stem cells used as disclosed here can be also derived from human embryonic germ (EG) cells. Human EG cells are prepared from the primordial germ cells obtained from human fetuses of about 8-11 weeks of gestation using laboratory techniques known to anyone skilled in the arts. The genital ridges are dissociated and cut into small chunks which are thereafter disaggregated into cells by mechanical dissociation. The EG cells are then grown in tissue culture flasks with the appropriate medium. The cells are cultured with daily replacement of medium until a cell morphology consistent with EG cells is observed, typically after 7-30 days or 1-4 passages. For additional details on methods of preparation human EG cells see Shamblott et al., [Proc. Natl. Acad. Sci. USA 95: 13726, 1998] and U.S. Pat. No. 6,090,622.

As mentioned hereinabove, the human stem cells as disclosed here may be also derived from a fetal or an adult source, such as for example, mesenchymal stem cells.

The phrase "mesenchymal stem cell" or "MSC" is used interchangeably for fetal or adult cells which are not terminally differentiated, which can divide to yield cells that are either stem cells, or which, irreversibly differentiate to give rise to cells of a mesenchymal cell lineage.

The mesenchymal stem cells as disclosed here may be of a syngeneic or allogeneic source.

Mesenchymal stem cells may be isolated from various tissues including, but not limited to, bone marrow, peripheral blood, blood, placenta and adipose tissue. A method of isolating mesenchymal stem cells from peripheral blood is described by Kassis et al [Bone Marrow Transplant. 2006 May;37(10):967-76]. A method of isolating mesenchymal stem cells from placental tissue is described by Zhang et al [Chinese Medical Journal, 2004, 117 (6):882-887]. Methods of isolating and culturing adipose tissue, placental and cord blood mesenchymal stem cells are described by Kern et al [Stem Cells, 2006;24:1294-1301].

Bone marrow can be isolated from the iliac crest of an individual by aspiration. Low-density BM mononuclear cells (BMMNC) may be separated by a FICOL-PAGUE density gradient. In order to obtain mesenchymal stem cells, a cell population comprising the mesenchymal stem cells (e.g. BMMNC) may be cultured in a proliferating medium capable of maintaining and/or expanding the cells. The populations may be plated on polystyrene plastic surfaces (e.g. in a flask) and mesenchymal stem cells are isolated by removing non-adherent cells. Alternatively mesenchymal stem cell may be isolated by FACS using mesenchymal stem cell markers. Examples of mesenchymal stem cell surface markers include but are not limited to CD105+, CD29+, CD44+, CD90+, CD34-, CD45-, CD19-, CD5-, CD20-, CD11B- and FMC7-. Other mesenchymal stem cell markers include but are not limited to tyrosine hydroxylase, nestin and H-NF.

Neural stem cells can also be used in accordance with the present invention. Makers such as Sox1, Sox2, SSEA-1/LeX can be used as possible markers for neural cell selection. Fine separation techniques based on negative FACS assay (in order to exclude lineage-restricted cells) and immunomagnetic beads may be used to obtain purified and homogeneous stem populations (Cai 2003 Blood Cells Mol Dis 31:18-27).

Stem cells used by the present invention can be also derived from induced pluripotent stem cells. Induced multipotent and pluripotent stem cell lines are referred to as "induced stem cell lines" (iSC lines) herein. Induced pluripotent stem cells are referred to as iPS cells or iPSCs.

Regardless of their origin, stem cells used in accordance with the present invention are at least 50 % purified, more preferably at least 75 % purified and even more preferably at least 90 % purified. When human embryonic stem cell lines are used, the human ES cell colonies are separated from their feeder layer (x-ray irradiated fibroblast-like cells) such as by mechanical and/or enzymatic means to provide substantially pure stem cell populations.

Once human stem cells are obtained, they may be treated to form aggregates.

Cells or preferably aggregates thereof are then subjected to differentiation conditions which comprise retinoic acids, such that neurospheres are allowed to form. The culture medium used is selected according to the stem cell used. Thus, for example, a medium suitable for ES cell growth, can be for example DMEM/F12 (Sigma- Aldrich, St. Lewis, MO) or alpha MEM medium (Life Technologies Inc., Rockville, MD, USA), supplemented with supporting enzymes and hormones. These enzymes can be for example insulin (ActRapid; Novo Nordisk, Bagsværd, DENMARK), progesterone and/or Apo transferring (Biological Industries, Beit Haemek, Israel). Other ingredients are listed in Example 1 of the Examples section.

As used herein the phrase "retinoic acid" refers to an active form (synthetic or natural) of vitamin A, capable of inducing neural cell differentiation. Examples of retinoic acid forms which can be used include, but are not limited to, retinoic acid, retinol, retinal, 11-cis-retinal, all-trans retinoic acid, 13-cis retinoic acid and 9-cis-retinoic acid (all available at Sigma- Aldrich, St. Lewis, MO).

Retinoic acid is preferably provided at a concentration range of 1-50 µM.

The culture medium may be further supplemented with growth factors which may be present at least in part of the culturing period to promote cell proliferation and facilitate differentiation into the neuronal glial lineages. Growth factors include for example EGF (10-40 ng/ml) and bFGF (10-40 ng/ml) (R&D Systems, Minneapolis, MN, Biotest, Dreieich, Germany).

As used herein the phrase "neurospheres" refers to quasi-spherical clusters or spheres containing mainly neural stem cells and early multipotent progenitors that can differentiate into neurons, oligodendrocytes and astrocytes as well as other glial cells.

The cells are cultured until ripened neurospheres are formed.

As used herein the phrase "ripened neurospheres" refers to neurospheres in which some of the neural stem cells have differentiated to become specialized oligodendrocyte progenitors having acquired makers of the oligodendrocyte lineage (e.g. Sox10, Nkx2.2., NG2, A2B5), while others have differentiated to become neural progenitors or astrocytes progenitors.

The cells may be allowed to culture for example for 10-30 (e.g., 20-30) days, at the end of which detached neurospheres are formed. The spheres, or cells dissociated therefrom, are then adhered to substrates and subjected to further expansion with growth factors and eventually to differentiation after removal of growth factors. Examples of adherent substrates which can be used in accordance with the teachings of the present invention include, but are not limited to matrigel or an extracellular matrix component (e.g., collagen, laminin and fibronectin).

Following neuroglial sphere formation (or concomitantly with) or following culture on adherent substrate, the cells are incubated with an agent capable of inhibiting Bone Morphogenetic Protein (BMP) activity.

Bone morphogenetic proteins (BMPs) are signaling molecules, belonging to the TGF-β superfamily, which act locally on target cells to affect cell survival, proliferation, and differentiation, and among other actions, regulate neural cell development.

Bone morphogenetic proteins (BMPs), were shown to inhibit oligodendrocyte development from rat fetal brain (Mehler et al., 1997; Mehler et al., 2000). The present inventors have found, as illustrated in the Examples section which follows, that the expression of several members of the BMP family, seen in undifferentiated human ES cells, increased or was even induced after culturing cells with retinoic acid. Expression of some BMPs continued to increase throughout the culturing steps. Since BMPs inhibit oligodendrocyte differentiation, the inhibition of these proteins is critical for inducing differentiation of that lineage.

As used herein the term "agent capable of down-regulating BMP activity" refers to an agent which can at least partially reduce the function (i.e., activity and/or expression) of BMP

BMP reducing agents include cystine knot-containing BMP antagonists, which are divided into three subfamilies, based on the size of the cystine ring; CAN (eight-membered ring), twisted gastrulation (nine-membered ring), and chordin and noggin (10-membered ring). The CAN family is divided further based on a conserved arrangement of additional cysteine residues, and includes gremlin and PRDC; cerberus and coco; DAN; USAG-1 and sclerostin.

Other BMP inhibitors include, but are not limited to, chordin like BMP inhibitor (CHL2), Neuralin (also homologous to chordin) which behave as secreted BMP-binding inhibitors; inhibin (belongs to the TGF-β superfamily); follistatin (which binds to inhibin); GDF3, an inhibitor of its own subfamily (TGF-β), which blocks classic BMP signaling in multiple contexts; Crossveinless-2 (hCV-2), a BMP function inhibitor; Ectodin (available at Qiagene, Valencia, CA), which is homologous to sclerostin and inhibits the activity of BMP2, BMP4, BMP6, and BMP7; connective tissue growth factor (CTGF), a BMP receptor antagonist; BMP-3, a BMP receptor antagonist; and gp130 signaling cytokines.

It will be appreciated that oligonucleotide inhibitors, which down-regulate expression of BMP genes (e.g., siRNA) may also be used in accordance with this aspect of the present invention. Methods of genetically modifying stem cells are well known in the art.

In one preferred embodiment of this aspect of the present invention the agent used to inhibit BMP is LDN-193189,.

As used herein, LDN-193189 is a cell permeable small molecule inhibitor of bone morphogenetic protein (BMP) type I receptors ALK2 and ALK3 (IC50 = 5 nM and 30 nM respectively). LDN-193189 was derived from structure-activity relationship studies of Dorsomorphin and functions primarily through prevention of Smad1, Smad5, and Smad8 phosphorylation. LDN-193189 is a hydrochloride salt

The phrase "myelination of neuronal cells" refers to the ability of oligodendrocytes (i.e. processes extending therefrom) to wrap around neuronal axons and form myelin sheaths.

As used herein, the term "maturation of OPCs" refers to the maturation or differentiation program of OPCs into myelinating oligodendrocytes.

In order to obtain populations of oligodendrocyte precursors that could be passaged and expanded before terminal differentiation, NS are plated on an adherent substrate and thereafter are subjected to one passage by for example, trypsinization to yield dissociated neuroglial sphere cells (huEs-NSc). These, can be further plated on cationic substrates for further culture and passaging, and then be subjected to terminal differentiation.

Examples of adherent substrates which can be used in accordance with the teachings of the present invention include, but are not limited to, cationic substrate which can be poly-D- lysine or Polyornithine with fibronectin (FN).

As shown in the Examples section which follows, cells can be split every 8-10 days for more than 3 passages, preferably more than 5 passages.

While reducing the present invention to practice, the present inventors have discovered that neurospheres (NS) cultured for 4 days on the adherent matrigel substrate formed a network of neurons, but these tended to disappear after prolonged culture on matrigel. Therefore, when culturing neurospheres for the purpose of producing neurons, inventors have found that culturing and passaging cells on an adherent substance which is not matrigel or the like, does not reduce neuron formation as observed for cells grown on matrigel.

Thus, to generate neurons, ripened NS are preferably plated on a cationic adherent substance and cultured with growth factors. In addition to BMP cultured cells can be passaged for more than 5 times, with the addition of growth factors in every passage.

In any of the above protocols laminin and vitamin C may be added to the culture , laminin being an extracellular matrix component which helps cells to adhere, and Vitamin C (ascorbic acid) being a well-known antioxidant.

Populations of cells generated according to the teachings of the present invention may comprise for example at least about 40 % of mature oligodendrocytes (comprising at least one mature oligodendrocyte phenotype as described above) and any where between 0-60 % cells which comprise a stem cell phenotype. Populations of oligodendrocyte precursors (OPC) may comprise at least 90% of bipolar O4⁺ cells (and about 10 % of cells having a stem cell phenotype).

Thus, as illustrated in the Examples section below, the cells differentiated according to the methods of the present invention represent a mature oligodendrocyte like shape, or oligodendrocyte like precursor shape and are accompanied by the presence of the appropriate oligodendrocyte marker.

The percentage of the cells of interest may be raised or lowered according to the intended needs. This may be effected by FACS using an antibody specific for a cell marker. Examples of such markers are described hereinabove. If the cell marker is an internal marker, preferably the FACS analysis comprises antibodies or fragments thereof which may easily penetrate a cell and may easily be washed out of the cell following detection. The FACS process may be repeated a number of times using the same or different markers depending on the degree of enrichment and the cell phenotype required as the end product.

Once differentiated and optionally isolated, the cells may be tested (in culture) for their phenotype. The cultures may be comparatively analyzed for a phenotype of interest (e.g., myelin production, expansion, migration), either in vitro and/or invivo using biochemical analytical methods such as immunostaining, cell expansion assays (e.g., MTT), migration assays, Western blot and Real-time PCR (some assays are described in Examples 1-5 of the Examples section which follows).

Cells generated according to the teachings of the present invention (described hereinabove) and in the Examples section which follows may be used in a myriad of clinical and research applications.

As used herein, the phrase "medical condition of the CNS" refers to any disorder, disease or condition of the central nervous system which may be treated with the cells of the present invention.

Accordingly, these cells can be used for preparing a medicament (interchangeably referred to as pharmaceutical composition), whereby such a medicament is formulated for treating a medical condition of the CNS.

Representative examples of CNS diseases or disorders that can be beneficially treated with the cells described herein include, but are not limited to, a pain disorder, a motion disorder, a dissociative disorder, a mood disorder, an affective disorder, a neurodegenerative disease or disorder, an injury, a trauma and a convulsive disorder.

For example, the cells may comprise oligodendrocyte cells and the medical condition can be selected from, for example, the group of autoimmune diseases, multiple sclerosis, Guillan-Barre syndrome or congenital leukodystrophies, adrenoleukodystrophies, Pelizaeus-Merzbacher, Charcot-Marie-Tooth, Krabbe or Alexander disease, vanishing white matter syndrome, progressive multifocal leukoencephalopathy, infectious demyelinating diseases, postinflammatory demyelinated lesions, neurodegenerative diseases, multisystem degeneration, vascular diseases, ischemic white matter damage, vascular leukoencephalopathies, subcortical infarcts, brain trauma, spinal cord trauma, demyelinative injury, neoplasms and oligodendrioglioma.

In this respect, it should be noted that oligodendrocyte precursor cells (OPCs) may be adventitiously used over mature oligodendrocytes as it is probably the mitotic and migratory capacity of these cells (in contrast to mature cells) which are vital prerequisites for successful remyelination. According to the present invention the remyelinating capacity of the OPCs is significantly enhanced when the OPC have been treated with LDN-193189 under the described conditions.

Mature differentiated oligodendrocytes may still be useful as myelinating cells in vivo (Duncan et al. 1992 Dev. Neurosci. 14:114-122). Differentiated and mature human oligodendrocytes may have important applications for testing drugs that can protect oligodendrocytes from toxic or other pathogenic injuries as further described hereinbelow.

The cells of the present invention can be administered to the treated subject using a variety of transplantation approaches, the nature of which depends on the site of implantation.

The term or phrase "transplantation", "cell replacement" "cell therapy" or "grafting" are used interchangeably herein and refer to the introduction of the cells of the present invention to target tissue. The cells can be derived from the recipient or from an allogeneic or xenogeneic donor, or from embryonic stem cells.

For example, the cells can be grafted into the central nervous system or into the ventricular cavities or subdurally onto the surface of a host brain or in the spinal cord. Conditions for successful transplantation include: (i) viability of the implant and sufficient number of cells; (ii) retention (e.g., astrocytes) or migration (e.g., OPCs) of the cells within the nervous tissue to the lesions in accordance with the selected population of cells; and (iii) minimum amount of pathological reaction. Methods for transplanting various nerve tissues, for example embryonic brain tissue, into host brains have been described in: "Neural grafting in the mammalian CNS", Bjorklund and Stenevi, eds. (1985); Freed et al., 2001; Olanow et al., 2003). These procedures include intraparenchymal transplantation, i.e. within the host brain (as compared to outside the brain or extraparenchymal transplantation) achieved by injection or deposition of tissue within the host brain so as to be opposed to the brain parenchyma at the time of transplantation.

Intraparenchymal transplantation can be effected using two approaches: (i) injection of cells into the host brain parenchyma or (ii) preparing a cavity by surgical means to expose the host brain parenchyma and then depositing the graft into the cavity. Both methods provide parenchymal deposition between the graft and host brain tissue at the time of grafting, and both facilitate anatomical integration between the graft and host brain tissue. This is of importance if it is required that the graft becomes an integral part of the host brain and survives for the life of the host.

Alternatively, the graft may be placed in a ventricle, e.g. a cerebral ventricle or subdurally, i.e. on the surface of the host brain where it is separated from the host brain parenchyma by the intervening pia mater or arachnoid and pia mater. Grafting to the ventricle may be accomplished by injection of the donor cells or by embedding the cells in a substrate such as 3% collagen to form a plug of solid tissue which may then be implanted into the ventricle to prevent dislocation of the graft. For subdural grafting, the cells may be injected around the surface of the brain after making a slit in the dura. Injections into selected regions of the host brain may be made by drilling a hole and piercing the dura to permit the needle of a microsyringe to be inserted. The microsyringe is preferably mounted in a stereotaxic frame and three dimensional stereotaxic coordinates are selected for placing the needle into the desired location of the brain or spinal cord. The cells may also be introduced into the putamen, nucleus basalis, hippocampus cortex, striatum, substantia nigra or caudate regions of the brain, as well as the spinal cord.

The cells may also be transplanted to a healthy region of the tissue. In some cases the exact location of the damaged tissue area may be unknown and the cells may be inadvertently transplanted to a healthy region. In other cases, it may be preferable to administer the cells to a healthy region, thereby avoiding any further damage to that region. Whatever the case, following transplantation, the cells preferably migrate to the damaged area.

For transplanting, the cell suspension is drawn up into the syringe and administered to anesthetized transplantation recipients. Multiple injections may be made using this procedure.

The cellular suspension procedure thus permits grafting of the cells to any predetermined site in the brain or spinal cord, is relatively non-traumatic, allows multiple grafting simultaneously in several different sites or the same site using the same cell suspension, and permits mixtures of cells from different anatomical regions. Multiple grafts may consist of a mixture of cell types, and/or a mixture of transgenes inserted into the cells. Preferably from approximately 10⁴ to approximately 10⁸ cells are introduced per graft.

For transplantation into cavities, which may be preferred for spinal cord grafting, tissue is removed from regions close to the external surface of the central nerve system (CNS) to form a transplantation cavity, for example as described by Stenevi et al. (Brain Res. 114:1-20., 1976), by removing bone overlying the brain and stopping bleeding with a material such a gelfoam. Suction may be used to create the cavity. The graft is then placed in the cavity. More than one transplant may be placed in the same cavity using injection of cells or solid tissue implants. Preferably, the site of implantation is dictated by the CNS disorder being treated.

For example, in treating multiple sclerosis transplantation into a small number of carefully chosen lesions, for example, the optic nerves, the spinal cord, or the superior cerebellar peduncle can be affected.

In other inherited disorders of myelin metabolism, a systemic mode of administration may be used to exploit the migratory capacity of OPCs and both the circulation of the brain and the blood. In these cases disruption of the blood-brain barrier and/or supplementation with growth factor infusion or growth/trophic factor secreting cells.

Since non-autologous cells are likely to induce an immune reaction when administered to the body, several approaches have been developed to reduce the likelihood of rejection of non-autologous cells. These include either suppressing the recipient immune system or encapsulating the non-autologous cells in immunoisolating, semipermeable membranes before transplantation.

Examples of immunosuppressive agents include, but are not limited to, methotrexate, cyclophosphamide, cyclosporine, cyclosporin A, chloroquine, hydroxychloroquine, sulfasalazine (sulphasalazopyrine), gold salts, D-penicillamine, leflunomide, azathioprine, anakinra, infliximab (REMICADE.sup.R), etanercept, TNF.alpha. blockers, a biological agent that targets an inflammatory cytokine, and Non-Steroidal Anti-Inflammatory Drug (NSAIDs). Examples of NSAIDs include, but are not limited to acetyl salicylic acid, choline magnesium salicylate, diflunisal, magnesium salicylate, salsalate, sodium salicylate, diclofenac, etodolac, fenoprofen, flurbiprofen, indomethacin, ketoprofen, ketorolac, meclofenamate, naproxen, nabumetone, phenylbutazone, piroxicam, sulindac, tolmetin, acetaminophen, ibuprofen, Cox-2 inhibitors and tramadol.

Human iPSC's cells that are "patient specific" and therefore would not be subject to immune reaction may be obtained by nuclear transfer in enucleated oocytes (so called therapeutic cloning). Alternatively, human ES cell banks may be used to find cells that are HLA matched to the patient. In addition, genetic engineering of the ES cells may be done to down-regulate histocompatibility antigens and reduce the risk of immune reaction. For this purpose, it is possible to use siRNA or some viral genes (Lee EM, Kim JY, Cho BR et al, Biochem.Biophys. Res Commun. 326, 825-835, 2005).

In any of the methods described herein, the cells can be administered either *per se* or, preferably as a part of a pharmaceutical composition that further comprises a pharmaceutically acceptable carrier.

As used herein a "pharmaceutical composition" refers to a preparation of one or more of the chemical conjugates described herein, with other chemical components such as pharmaceutically suitable carriers and excipients. The purpose of a pharmaceutical composition is to facilitate administration of a compound to a subject.

Hereinafter, the term "pharmaceutically acceptable carrier" refers to a carrier or a diluent that does not cause significant irritation to a subject and does not abrogate the biological activity and properties of the administered compound. Examples, without limitations, of carriers are propylene glycol, saline, emulsions and mixtures of organic solvents with water.

Herein the term "excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

The pharmaceutical carrier may be an aqueous solution of saline.

Techniques for formulation and administration of drugs may be found in "Remington's Pharmaceutical Sciences," Mack Publishing Co., Easton, PA, latest edition.

Suitable routes of administration include direct administration into the tissue or organ of interest. Thus, for example the cells may be administered directly into a specific region of the brain or to the spinal cord.

For any preparation used in the methods of the invention, the therapeutically effective amount or dose can be estimated initially from *in vitro* and cell culture assays. Preferably, a dose is formulated in an animal model to achieve a desired concentration or titer. Such information can be used to more accurately determine useful doses in humans.

Toxicity and therapeutic efficacy of the active ingredients described herein can be determined by standard pharmaceutical procedures *in vitro,* in cell cultures or experimental animals. For example, animal models of demyelinating diseases include shiverer (shi/shi, MBP deleted) mouse, MD rats (PLP deficiency), Jimpy mouse (PLP mutation), dog shaking pup (PLP mutation), twitcher mouse (galactosylceramidase defect, as in human Krabbe disease), trembler mouse (PMP-22 deficiency). Virus induced demyelination model comprise use if Theiler's virus and mouse hepatitis virus. Autoimmune EAE is a possible model for multiple sclerosis.

The data obtained from these *in vitro* and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition, (see e.g., Fingl, et al., 1975, in "The Pharmacological Basis of Therapeutics", Ch. 1 p.1). For example, Parkinson's patient can be monitored symptomatically for improved motor functions indicating positive response to treatment.

For injection, the active ingredients of the pharmaceutical composition may be formulated in aqueous solutions, preferably in physiologically compatible buffers such as Hank's solution, Ringer's solution, or physiological salt buffer.

Dosage amount and interval may be adjusted individually to levels of the active ingredient which are sufficient to effectively regulate the neurotransmitter synthesis by the implanted cells. Dosages necessary to achieve the desired effect will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or diminution of the disease state is achieved.

The amount of a composition to be administered will, of course, be dependent on the individual being treated, the severity of the affliction, the manner of administration, the judgment of the prescribing physician, etc. The dosage and timing of administration will be responsive to a careful and continuous monitoring of the individual changing condition. For example, a treated Parkinson's patient will be administered with an amount of cells which is sufficient to alleviate the symptoms of the disease, based on the monitoring indications.

The cells of the present invention may be co-administered with therapeutic agents useful in treating neurodegenerative disorders, such as gangliosides; antibiotics, neurotransmitters, neurohormones, toxins, neurite promoting molecules; and antimetabolites and precursors of neurotransmitter molecules such as L-DOPA. Additionally, the cells of the present invention may be co-administered with other cells.

Following transplantation, the cells of the present invention preferably survive in the diseased area for a period of time (e.g. at least 6 months), such that a therapeutic effect is observed. As described in Example 4, the cells of the present invention were shown to migrate myelinate in shiverer mouse brain.

Generally, any method known in the art can be used to monitor success of transplantation. For example, MRI can be used for visualizing brain white matter and studying the burden of delyelinating lesions as currently practiced for monitoring MS patients. Magnetization transfer contrast can be used to monitor remyelination (Deloire-Grassin 2000 J. Neurol. Sci. 178:10-16). Magnetic resonance spectroscopy measurement of N-acetyl-aspartate levels can be used to assess impact on local neuron/axon survival. Using paramagnetic particles to label cells before transplantation enabling their dispersion to be tracked by MRI Serial neurophysiology is useful for monitoring conduction. The optic nerve has particular advantages in this respect.

Other approaches to more generalized neurophysiological assessment are described in Leocani et al. Neurol Sci. 2000;21(4 Suppl 2):S889-91 which may be useful for interventions aimed at multifocal or more diffuse myelin repair. Notwithstanding, it is appreciated that clinical improvement may also be assessed. Demyelination causes alterations of stature (trembling, shivering) and locomotion. Children with leukodystrophies have motor and intellectual retardation. Electrophysiological measures of sensory and motor nerve conductivity, for example H-wave, are classical method used in monitoring neuropathies linked to demyelinating peripheral lesions (Lazzarini et al, eds (2004) Myelin biology and disorders, Elsevier Academic Press, San Diego, CA).

As mentioned above, cells of the present invention can be used as an imperative tool for in vitro screening of drugs.

Thus, according to yet another aspect of the present invention there is provided a method of determining an effect of a drug on neural cell functionality, the method comprising subjecting a cell of the present invention (e.g., oligodendrocyte) to the drug and determining at least one of a structural or functional phenotype of the treated cell as compared to an untreated cell, thereby determining an effect of the drug on neural cell functionality.

Qualifying the effect of a drug of interest on the cells of the present invention can be used to identify and optimize treatments capable of restoring the neural function, and hence can be used to identify and optimize drugs suitable for treating neural disorders (e.g., including treatment methods envisaged by the present invention).

Furthermore, qualifying the effect of a drug (either directed to diseases of the CNS or any other tissue) on neural functionality can be used to assess the toxicity of clinical drug applications.

Thus, this aspect of the present invention can be preferably utilized to determine the therapeutic and toxic effects of various drugs.

Hence the method of the present invention can be used to screen and/or test drugs.

This aspect of the present invention can be also utilized to obtain gene expression profiles and changes thereof in cells of the present invention subjected to a drug. Thus, the method according to this aspect of the present invention can be used to determine, for example, gene expression pattern changes in response to a drug.

As used herein the term "about" refers to ± 10 %.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions; illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### EXAMPLE 1

### Proliferation and freezing of glial progenitor cells

Differentiation of human ES cells to glial progenitors was conducted as described (Izrael et al, 2007, Mol Cell Neurosci. 34(3):310-23). About 200 Neurospheres resulting from differentiation in suspension for 25 days were seeded per well of Matrigel coated 6 well plates, without dissociation (passage zero) (Matrigel BD, without growth factor, diluted 1/30). Neurospheres grown on Matrigel for one to two weeks were dissociated with Trypsin 0.025%-EDTA (Gibco/Life Technologies) diluted 10xfold in PBS (minus Ca++ and Mg++, PBS--) (Gibco/Life Technologies) (passage 1). Cells were washed in 37°C PBS--, 3ml/well of 6 well plate. Trypsin diluted, 800µl per well, was left on plate for 3 min, and defined trypsin inhibitor (DTI) 800µl was added to the same well. After careful shaking to the side of the plate to detach cells, 2ml of 2% BSA (Sigma, A4919) in DMEM/F12 (Gibco/Life Technologies) with 1% B27 and 0.5% N2 supplements were added (Gibco/Life Technologies). After centrifugation for 6 min at 1200 rpm (280xg), the pellet was suspended in complete growth medium (GM: DMEM/F12 (Biological Industries, bet ha Emek Israel) with 1% B27 and 0.5% N2 supplements, 10ng/ml bFGF (R&D) and 10ng/ml EGF (R&D), penicillin/streptomycin (Biological industries, Bet ha Emek Israel), Glutamax (Gibco/life technologies)), and cells dispersed (by careful pipetting if necessary) before seeded again on Matrigel at the density of 50,000 cells/cm2. After 7 days the cells were dissociated from the plate as above and reseeded on Matrigel for 7 days (passage 2). The cells at passage 2 grown for 7 days were dissociated and frozen (passage 3). The cell pellet after dissociation with trypsin and centrifugation was suspended in complete growth medium (one volume) and freezing medium (one volume). The freezing medium contained 15% DMSO and 60% knock out serum replacement (SRKO, Gibco/Life Technologies), 25% complete growth medium (GM). Each ampule contained 1-2x10^6 cells.

### EXAMPLE 2

### Differentiation of glial progenitor cells (GPC) into oligodendrocytes and astrocytes

Glial progenitor cells proliferation took place in GM medium using 6 well or 175CM^2 flaks. In order to initiate differentiation 20,000 GPCs were seeded in each well of Matrigel coated plates of 96 wells, or on PolyD-Lysine/laminin (PDL/lam)- coated 96 well plates. Cells were grown in GM media for two to three days. Then GM was replaced by differentiation medium without growth factors, (DMEM/F12, 1% B27, 0.5% N2, Vitamin C (Sigma, 50ug/ml). Differentiation medium was supplemented with the following tested compounds: LDN 193189 (STEMGENT) in two concentrations (62.5nM and 125nM), 100ng/ml Noggin (R&D), 2µM Dorsomorphin (STEMGENT) and 20ng/ml human recombinant PDGFAA (R&D). In order to extract the EC50, a dose response assay was done supplementing the cells with LDN-193189 (STEMGENT) at concentrations of 2uM to 0.0075 uM with two fold dilutions over ten wells in triplicate. All media were replaced every other day.

### Preparation of PDL

PDL (Sigma P6407) was dissolved into water (200µg/ml), then neutralized with Borate buffer pH 8 and diluted to 100 µg/ml. The PDL solution is distributed into 96 well plates 50ul per well and left 4 hours to overnight in the incubator at 37°C. The PDL was washed 3 times with 170ul sterile water and left at 4°C until use. Before using laminin (20ug/ml in GM) was added to each well and was left 2-4 hours at 37°C.

### EXAMPLE 3

### Effect of the Alk Kinase inhibitor, LDN-193189 on the terminal differentiation of human oligodendrocytes derived from ES cells

### MATERIALS AND EXPERIMENTAL PROCEDURES

### Immunostaining

Oligodendrocytes were characterized by the presence of the antigen detected by the monoclonal antibody O4 (IgM (R&D). Living cells reacted with O4 antibody diluted 1/200 in medium without growth factors (50ul/well) and left for 40 minutes in the incubator at 37°C, washed once with GM, fixed with PFA 4% (170ul per well) for 20 minutes at room temperature, washed 3 times with PBS. Dapi diluted to 0.5ug/ml was introduced in the second wash for 10 min. Afterwards the preparation was blocked with 5% normal goat serum in PBS-- for 20 min, washed and reacted with goat anti mouse IgM Ab-FITC conjugated (Millipore cat AP500F) diluted 1/500, for 45 minutes at room temperature, and washed three times with PBS--.

The oligodendrocyte lineage cells were characterized by the expression of a transcription factor olig2. The rabbit polyclonal AB anti olig2 (Millipore cat AB9610) was added to PFA fixed cells. The blocking solution contained 1% BSA and 0.5% normal goat serum and 0.25% Triton X100, and left for 20 min at RT. The fixed cells were washed three times in blocking solution, olig2 diluted 1/500, in blocking solution, was added for 1 hour. The fixed cells were washed with PBS 3 times and goat anti rabbit Alexa Fluor 568 1/500 in PBS was added for 45 minutes. The fixed cells were washed three times.

### FACS Analysis

Single cells were centrifuged and re-suspended in PBS-0.5% Bovine serum albumin, and were reacted for 15 minutes at 4°C with Ab (10µl/100ul for CD140 or O4, Miltenyi Biotec) or 5 µl Ab (A2B5, Miltenyi Biotec). The cells were washed with 1ml PBS, centrifuged at 300xg for 5 min and re-suspended in 100 µl PBS. Flow cytometry was analyzed in BDFACS canto machine.

### High content screening analysis

ArrayScan VTI HCS reader system (Cellomis, Thermo-scientific) was used for scanning at least 20 fields for each tested well under 10X objective in both assays (differentiation and myelination). Cellomics Neural profiling bio-application was used for processing and analyzing the images. For differentiation assay, the following parameters were tested: number and percentage of O4+ cells, number of processes, processes length and number of branching points. For myelination assay, the same morphological parameters were tested and in addition: areas of myelinated axons in pixels (MBP&NF overlap area) and Number of myelinated regions (MBP&NF overlap count) were also analyzed. Modifications in the Neuro-profiling bio-application were conducted for this specific purpose. T-test analysis was used to compare between treatments. The 4-parameter Hill-Slope curve fittings were generated by GraphPad Prism software using least squares fitting method and EC50 values were calculated.

### Co-culture myelination assay

### Mouse or rat DRG neurons purification

DRG dissociated cells from mouse embryonic day 13 (E13) or rat embryonic day (E15) were seeded on matrigel and PDL-coated 96 well plates (20, 000 cells/well). In order to allow long culture and co-culture of neuron axons in 96 well plates without de-attachment, a method where the periphery of each well was scratched leaving sticky edges (and smooth surface) was developed. This scratch methodology enables long lasting culture of neuron axons on 96 well format. Once DRG neurons were seeded they were fed with Neurobasal medium (Gibco) supplemented with B27 (Gibco) and 50 ng/ml NGF (Alomone labs) (NB medium) for 2-3 weeks prior hES-OPC addition. At the first week of DRG culture, the cells were treated with two cycles (2 days each) of NB medium containing 10 µM uridine/10 µM 5'-Fluoro 2'-deoxyuridine (Sigma) to eliminate fibroblasts and Schwann cells.

In the co-culture myelination assay, cells were fixed using 4% paraformaldehyde (PFA), washed twice with 1x PBS and kept at 4°C before staining. Non-specific staining was blocked with normal goat serum (5% w/v in PBS) and 0.3% Triton (TX-100) was used for cell permeabilization for 30 minutes at RT. Cells were washed twice with 1x PBS. For Oligodendrocytes myelin staining, the cells were incubated with Rat anti Myelin basic protein (MBP) (Abcam) for 1hr, washed twice with 1xPBS. Then cells were incubated with goat anti-rat IgG-Cy3 (Jackson Lab) for 30 min and washed twice. For staining neural axons, cells were incubated with rabbit polyclonal anti Neurofilament (Abcam) for one hour, and then washed twice with 1 x PBS. Immediately after, cells were incubated with Alexa Fluor® 488 goat anti&rabbit (Invitrogen) for 30 minutes, and washed twice with 1xPBS. Following MBP, NF and DAPI staining (Sigma) used for nuclear staining.

### RESULTS

Effect of LDN-193189 on human oligodendrocyte maturation was examined by direct visualization and counting of immunostained cells cultured with and without the addition of LDN-193189. As shown in FIGs. 1A-B, Noggin and LDN-193189 stimulate the conversion of neural stem cells to Olig2 positive cells with the same kinetics. Figure 1A shows that Neural Stem Cells (NSCs) at passage five prior to the addition of differentiation medium contained very low percentage of cells of the oligodendrocyte lineage (olig2 positive or PDGFRA positive). The cells at passage five were cultured for three days with GM on 96 well plates and after fixation, cells were reacted with anti-olig2 Ab, and olig2 positive cells and nuclei were scanned in the Thermo Scientific™ ArrayScan™ XTI High Content Analysis (HCA) Reader and counted with the neural profiling program. On the other hand the cells surface antigens O4 and PDGFRa were detected on dissociated cells after fixation and reaction with O4 Ab conjugated to APC, anti PDGFRa PE, as well as A2B5 PE all obtained from BD. Flow cytometry was conducted in BD FACS Canto II. Figure 1B demonstrates the comparison between the number of olig2 positive cells in non-treated or treated with PDGF, Noggin or LDN-193189 alone. Time points used for analysis are 21, 28, 35 and 42 days of differentiation, for each time point 20 fields were analyzed. Cells at passage 5 were seeded on 96 well plates and after 4 days in GM, medium was changed to differentiation medium without noggin (non-treated, (NT) cells), or with addition of noggin (100ng/ml), with 20ng/ml PDGF or with LDN-193189 at two concentrations (62.5nM and 125nM).

To further assess the dose response of LDN-193189 on development of mature oligodendrocytes, EC50 was calculated. FIG. 2 depicts LDN-193189 dose-response curves which show a plateau of activity at 62.5 to 125 nM and EC50 at about 30nM. Cells at passage 5 were cultured in 96 well plates in GM for 4 days, then treated with LDN-193189 from 2µM to 0.0075uM in differentiation medium without noggin, with or without PDGF (20ng/ml) for 21 days. In this experiment, cells at passage 4 were pretreated in differentiation medium with Noggin for 14 days, and split before seeding on 96 well plates. In spite of the cells being pretreated with noggin, the number of olig2 positive cells increases more with LDN-193189 and a typical curve of dose response with a plateau was obtained for the percentage of olig2 cells. The addition of PDGF during the assay increases the number of olig2 positive cells about two folds. The concentration giving half the maximal effect (EC50) starting from the same data were calculated and it was found to be around 30nM, with or without PDGF.

### EXAMPLE 4

### Opposite effects of Dorsomorphine and its LDN derivative on the terminal differentiation of human oligodendrocytes

Since Dorsomorphin is a molecule from which LDN-193189 was derived by chemical modification and is also an inhibitor of the same Alks, its effect on the terminal differentiation of human oligodendrocytes was tested in comparison to LDN and Noggin.

As shown in Figure. 3B, an increase in the number of O4+ cells (green) on day 21 of differentiation in Noggin+PDGF and LDN-193189 treatments (column 2 and 4 respectively) was detected, whereas Dorsomorphin and LDN+Dorsomorphin abolished hOPC differentiation (Column 3 and 5 respectively). Figure 3C demonstrates the kinetics (day 7, 21, 28 and 35) of hOPC differentiation as was measured by of O4+ cells count. From day 21 Z' factor was calculated (presented on graph). Noggin&PDGF and LDN presented valid Z'factor (Z'>0.25) in most of tested time points indicating a significant difference (P<0.0001) and presenting LDN-193189 as a good positive control for drug screening purposes. Figure 3D shows O4 staining (green) and DAPI (blue). Enlargement (inset) of representative O4+ cell with myelin sheaths (arrow) is presented in Noggin+PDGF and LDN-193189 treatment images.

As shown in Figure 4A. Noggin+PDGF and LDN-193189 treatments increased the number of Olig2+ (column 2 and 4 respectively), whereas LDN+Dorsomorphin decreased hOPC commitment (Column 5). Figure 4B demonstrates that the percentage of Olig2+, Noggin+PDGF and LDN-193189 significantly increased the number of Olig2+ cells (P<0.01), whereas LDN+Dorsomorphin decreased hOPC commitment when compared to LDN-193189.

For testing maturation and myelination capacity of the hPSC derived oligodendrocytes, a co-culture system between rodent dorsal root ganglia (DRGs) and hPSCs derived oligodendrocytes was used. The number of MBP+ cells (MBP, a marker of oligodendrocytes' myelin) and Myelin formation (measured by co-localization area between MBP and neurofilament positive neuronal axons) was quantified. As shown in FIGs. 5A-F, LDN-193189 increased the number of MBP+ cells.

Taken together, these results established that LDN-193189 increased the number of O4+ and Olig2+ cells, the number of processes per cell and total length of processes in a dose dependent manner. Surprisingly, Dorsomorphin, a BMP inhibitor that was used to derive LDN molecule was found to strongly inhibit Olig2+ cells differentiation and their conversion into O4+ cells. Similarly to results obtained by hOPC differentiation assay, it was found that LDN--193189 increased also the number of MBP+ cells, whereas Dorsomorphin abolished the differentiation into MBP+ cells. Dorsomorphin also affects the activity of other kinases, indicating involvement of other kinases in oligodendrocyte development.

### EXAMPLE 5

### Evaluation of LDN-193189 effects on EAE development in C57BL/6 mice

Experimental autoimmune (allergic) encephalomyelitis (EAE) is considered to be the best model of multiple sclerosis (MS), a chronic, often disabling disease of the human central nervous system (CNS). EAE is characterized by immune responses against CNS tissue and can be induced in animals by immunizing them against proteins of CNS.
Mice are immunized with MOG₃₅₋₅₅ peptide or MOG₁₋₁₂₅ emulsified in complete Freund's adjuvant (CFA) by injecting them subcutaneously at two sites on the back (0.1 mL of emulsion/site). On the same day, and again the following day, mice are receiving intraperitoneal injection of pertussis toxin in PBS, at up to 600 ng/mouse/dose (0.1 mL). EAE is developed in mice 7-14 days after immunization (Day 0). Animals which develop EAE will become paralyzed. Paralysis is usually chronic, with the most severe paralysis lasting 2-4 days.
Daily observation and scoring of mice starts on Day 7 and continues until the end of the study.

### EAE Scoring

Scoring is on the scale of 0 to 5, according to Table 1. Mice may be given "in-between" scores (i.e. 0.5, 1.5, 2.5, 3.5) when the clinical picture lies between two defined scores.

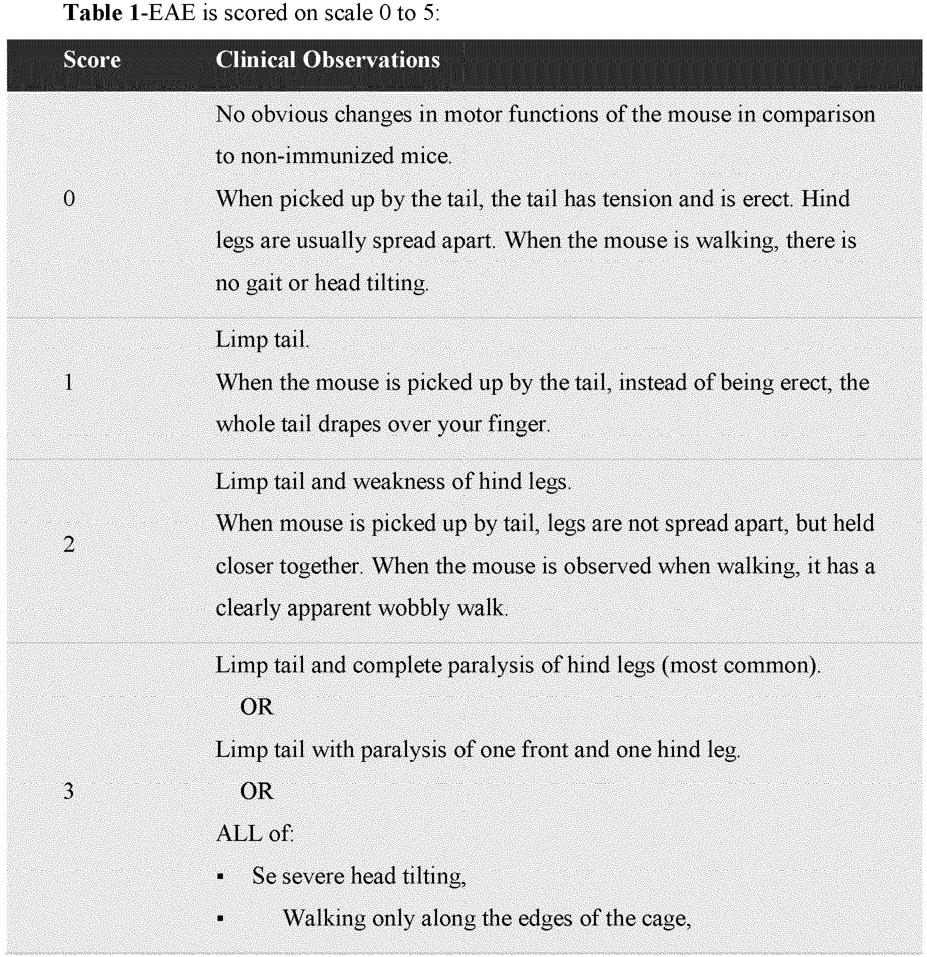

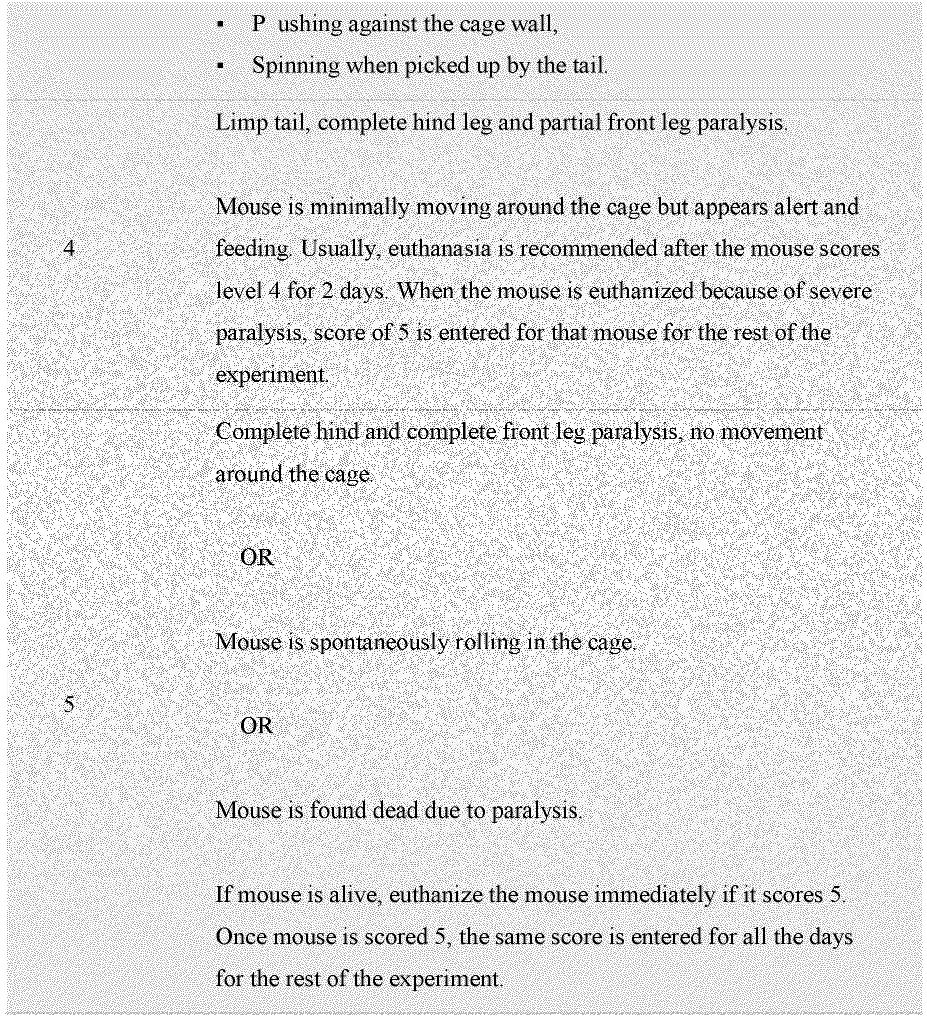

Mice are given "in-between" scores (i.e. 0.5, 1.5, 2.5, 3.5) when the clinical picture lies between two defined scores. In most cases mice reach clinical score of 3.5-4 and their clinical signs are improving from that point forward.

### Criteria used to euthanize mice

If an animal scores 5, it is immediately euthanized.

If an animal is severely paralyzed (score 4 or 4.5), it will be given s.c. fluid - 1 mL of 0.9% NaCl or Ringer's solution - and will be re-evaluated at the same time (+/- 1 hour) the following day. If that animal scores 4 or higher again, it will be euthanized immediately afterwards. This is done to ensure that no animal spends more than 24 hours with a score of 4 or higher.

In addition to EAE scoring, overall clinical appearance of the mice will be used as a criterion to euthanize mice. This criterion may override the EAE scoring criterion.

Blood collection: mice are bled during the experiment, approximately 0.1 to 0.2 mL of blood are collected from the retro-orbital plexus under complete anesthesia (isofluorane). The sample is used to analyze cytokine levels or leukocyte levels in blood and/or concentration of a therapeutic compound in plasma or serum.

For major terminal surgery, deep surgical anesthesia is achieved using i.p. administration of tribromoethanol. This is done when CNS tissue needs to be collected for immunohistochemistry. Once mice are fully anesthetized, as tested by absence of any reflex withdrawal of the leg after firm pinching of the foot with forceps, perfusion is performed, first with 5 to 10 mL of PBS, and then with 10 to 20 mL of 4% paraformaldehyde. The tissue is collected.

At the end of the study, animals are euthanized using CO₂ asphyxiation.

### LDN-193189 Treatment

Mice will receive LDN-193189 or vehicle on day 10-14 after EAE induction. The effect of LDN is tested in few routes of administration:
- Oral gavage - up to twice/day, up to 10 mL/kg, using a 1 inch long 25G gavage needle
- Intraperitoneal injection - up to once/day, up to 3 mL/kg
- Or combination

Animals are scored according to the score scale above and the effect of LDN treatment is evaluated.
Once the animals will reach 4-5 score they will be sacrificed and perfused. Brain and spinal cord specimen will be used frozen sectioned and stained for oligodendrocytes marker (i.e. O4, MBP, PLP, Olig2) in order to evaluate the effect of LDN-193189 on oligodendrocytes precursor cells development and functionality.

The experimental outline of in-vivo efficacy experiment for the effect of LDN on re-myelination is shown in Table 2. Four experimental groups are tested; Cohort 1- sham injected with physiological saline, Cohort 2- I.P injection of 1mg/ml LDN, Cohort 3-I.P injection of 3mg/ml LDN and Cohort 4- I.P injection of 9 mg/ml LDN. All injections are conducted daily. Histology and electron microscopy analysis are performed on day 30, 55 and 110 after EAE was induced on 10-12 week old Biozzi ABH female mice.

**Table 2-Experiment outline (Female 10-12w -Biozzi ABH EAE mouse models -1mg S.C injection of SC homogenate supplemented with 60mg mycobacterium tuberculosis)**

| | Cohort # | Cohort 1 | Cohort 2 | Cohort 3 |
|---|---|---|---|---|
| Treatment (I.P injection) 30d post EAE induction | Sham injected | LDN-1mg/kg | LDN-3mg/kg | LDN-9mg/kg |
| No. Animals | 13+3+4 | 13+3+4 | 13+3+4 | 13+3+4 |
| Injection | Daily | Daily | Daily | Daily |
| Analysis | Neurological score | Neurological score | Neurological score | Neurological score |
| Histology (Day 30 1^{st} remission (n=3), day 55 progressive (n=4), day 110 start of chronic phase (n=13)) | | | | |
| | Remyelination-EM&IHC | Remyelination-EM&IHC | Remyelination-EM&IHC | Remyelination-EM&IHC |
| ***EXAMPLE** 6* | 9 weeks monitoring from treatment | 9 weeks monitoring from treatment | 9 weeks monitoring from treatment | 9 weeks monitoring from treatment |

### EXEMPLE 6

### Gene expression hierarchical clustering of the effect of LDN on human oligodendrocytes cells.

LDN (125nM) was supplemented to human oligodendrocytes precursor cells (hOPC) for 35 days. Microarray analysis was performed on duplicate of LDN treated and Non-treated (NT) hOPC. LDN up regulated genes in human oligodendrocyte cell population are shown in Table 3. Bolded italic marked genes are known to take place in promoting oligodendrocytes maturation.

**Table 3- LDN up regulated genes**

| Transcript Cluster ID | LDN | NT | Fold Change LDN vs. NT | ANOVA p-value (LDN vs.NT) | Gene Symbol |
|---|---|---|---|---|---|
| 16808340 | 5.12 | 3.72 | 2.62 | 0.046492 | CATSPER2P1 |
| 16696624 | 8.09 | 6.92 | 2.24 | 0.02951 | **TNR** |
| 16970001 | 8.99 | 7.95 | 2.06 | 0.003011 | **UGT8** |
| 16797929 | 5.86 | 4.84 | 2.02 | 0.005189 | |
| 16800268 | 4.08 | 3.09 | 1.98 | 0.036668 | 0.036668CKMT1A |
| 16758848 | 4.29 | 3.33 | 1.95 | 0.006271 | |
| 16685729 | 4.53 | 3.57 | 1.94 | 0.009335 | BMP8B |
| 16985206 | 3.41 | 2.45 | 1.94 | 0.00916 | |
| 17056553 | 4.16 | 3.21 | 1.94 | 0.001649 | OTTHUMG000001 56244 |
| 16688992 | 5.8 | 4.86 | 1.92 | 0.048146 | LPAR3 |
| 17042403 | 7.47 | 6.53 | 1.92 | 0.000891 | |
| 16724356 | 4.18 | 3.27 | 1.87 | 0.0077 | MIR3160-1 |
| 16927827 | 5.34 | 4.45 | 1.86 | 0.014009 | IGLV3-9 |
| 16804141 | 3.87 | 2.99 | 1.85 | 0.006023 | EFTUD1P1 |
| 16662690 | 3.02 | 2.14 | 1.84 | 0.035876 | |
| 17112263 | 2.9 | 2.03 | 1.83 | 0.048112 | |
| 16766940 | 6.76 | 5.91 | 1.81 | 0.020941 | |
| 16997369 | 4.3 | 3.46 | 1.8 | 0.028645 | |
| 16863963 | 4.05 | 3.22 | 1.78 | 0.028755 | CGB2 |
| 16880712 | 8.96 | 8.13 | 1.78 | 0.000937 | SLC1A4 |
| 16674240 | 3.05 | 2.24 | 1.76 | 0.003487 | TEX35 |
| 16703561 | 5.3 | 4.49 | 1.76 | 0.042208 | |
| 16934881 | 6.63 | 5.81 | 1.76 | 0.034484 | **SOX10** |
| 16740950 | 5.53 | 4.73 | 1.74 | 0.026668 | |
| 16926511 | 5.66 | 4.86 | 1.74 | 0.038541 | |
| 17004061 | 3.13 | 2.33 | 1.74 | 0.0006 | OR2V1 |
| 16707466 | 3.92 | 3.12 | 1.73 | 0.023036 | |
| 16874641 | 5.05 | 4.28 | 1.71 | 0.023592 | SNORD88B |
| 16834632 | 3.21 | 2.45 | 1.69 | 0.008125 | |
| 16912470 | 2.92 | 2.16 | 1.69 | 0.026695 | RNA5SP482 |
| 16826102 | 5.02 | 4.28 | 1.67 | 0.026332 | OTTHUMG000001764 66 |
| 16909695 | 3.3 | 2.56 | 1.66 | 0.014935 | LOC100286922 |
| 16920475 | 6.52 | 5.8 | 1.65 | 0.0204 | BCAS1 |
| 16940440 | 4.98 | 4.26 | 1.65 | 0.003003 | MIR1226 |
| 17056789 | 6.64 | 5.92 | 1.65 | 0.040554 | |
| 16731401 | 4.26 | 3.55 | 1.64 | 0.042165 | CLDN25 |
| 16822561 | 5.67 | 4.95 | 1.64 | 0.037105 | MSLNL |
| 16846798 | 5.15 | 4.43 | 1.64 | 0.00836 | |
| 17063571 | 2.81 | 2.1 | 1.63 | 0.009191 | |
| 16697217 | 3.02 | 2.33 | 1.62 | 0.011376 | |
| 16782032 | 3.99 | 3.29 | 1.62 | 0.035399 | TRAJ46 |
| 16660753 | 3.33 | 2.64 | 1.61 | 0.040612 | LOC284632 |
| 16924761 | 7.51 | 6.83 | 1.61 | 0.017766 | GRIK1 |

LDN down regulated genes in human oligodendrocyte cell population are shown in Table 4.

**Table 4- LDN down regulated genes**

| Transcript Cluster ID | LDN | NT | Fold Change LDN vs. NT | ANOVA p-value (LDN vs.NT) | Gene Symbol |
|---|---|---|---|---|---|
| 16838101 | 3.01 | 3.71 | -1.62 | 0.015388 | SNORD1B |
| 16867229 | 3.63 | 4.33 | -1.62 | 0.004827 | SNORD37 |
| 16906256 | 3.32 | 4.02 | -1.62 | 0.028939 | |
| 17105179 | 3.47 | 4.17 | -1.62 | 0.027317 | MIR1321 |
| 16898108 | 2.38 | 3.09 | -1.63 | 0.024132 | SNORA70B |
| 16873632 | 6.29 | 7.01 | -1.64 | 0.019882 | SLC1A5 |
| 17028423 | 2.38 | 3.1 | -1.64 | 0.0139 | HCG24 |
| 17118266 | 2.43 | 3.14 | -1.64 | 0.044132 | |
| 16683377 | 5.5 | 6.22 | -1.65 | 0.039459 | **ID3** |
| 16755712 | 3.1 | 3.82 | -1.65 | 0.008589 | ANO4 |
| 16716499 | 3.56 | 4.29 | -1.66 | 0.024152 | NUDT9P1 |
| 16848961 | 5.41 | 6.15 | -1.67 | 0.026427 | TRIM65 |
| 16850724 | 3.09 | 3.84 | -1.67 | 0.022459 | ARHGAP28 |
| 17010552 | 5.68 | 6.43 | -1.67 | 0.040435 | TTK |
| 17124590 | 2.62 | 3.36 | -1.67 | 0.005145 | |
| 16781352 | 4.74 | 5.49 | -1.68 | 0.021886 | |
| 16966801 | 4.62 | 5.37 | -1.68 | 0.014149 | GSX2 |
| 16886491 | 5.47 | 6.23 | -1.69 | 0.037903 | TNFAIP6 |
| 16899533 | 3.97 | 4.74 | -1.7 | 0.02242 | LRRTM1 |
| 17095887 | 9.23 | 10 | -1.71 | 0.04131 | ASPN |
| 17117590 | 5.68 | 6.46 | -1.72 | 0.021424 | |
| 17077073 | 2.85 | 3.65 | -1.73 | 0.004019 | ST18 |
| 16661495 | 2.28 | 3.1 | -1.75 | 0.004332 | SCARNA1 |
| 16697040 | 3.18 | 3.99 | -1.76 | 0.007285 | |
| 16787902 | 5.07 | 5.89 | -1.76 | 0.031474 | SERPINA3 |
| 16928859 | 2.2 | 3.01 | -1.76 | 0.030934 | |
| 16911917 | 6.13 | 6.97 | -1.78 | 0.015035 | INSM1 |
| 16773183 | 3.69 | 4.53 | -1.79 | 0.043783 | C1 QTNF9B-AS 1 |
| 16941871 | 4.55 | 5.39 | -1.79 | 0.041837 | IL17RB |
| 16718670 | 7.52 | 8.38 | -1.82 | 0.001262 | GFRA1 |
| 16737325 | 6.08 | 6.94 | -1.82 | 0.026688 | SLC1A2 |
| 16979339 | 4.13 | 5 | -1.83 | 0.031408 | PDE5A |
| 16665976 | 3.48 | 4.36 | -1.84 | 0.004237 | LRRC7 |
| 16880774 | 3.13 | 4.01 | -1.84 | 0.049595 | FLJ16124 |
| 16924620 | 4.59 | 5.5 | -1.87 | 0.005818 | ADAMTS5 |
| 17012233 | 3.53 | 4.44 | -1.88 | 0.019931 | |
| 16905108 | 6.25 | 7.18 | -1.9 | 0.042976 | DLX2 |
| 17012223 | 4.53 | 5.46 | -1.9 | 0.033216 | CLVS2 |
| 16803754 | 4.07 | 5.01 | -1.92 | 0.032913 | KIAA1199 |
| 16689704 | 2.34 | 3.31 | -1.96 | 0.046685 | |
| 17013126 | 5.42 | 6.39 | -1.96 | 0.031286 | GPR126 |
| 16855510 | 3.98 | 4.99 | -2.02 | 0.018404 | ATP8B1 |
| 17044956 | 5.13 | 6.15 | -2.03 | 0.018194 | PPP1R17 |
| 16767081 | 5.73 | 6.78 | -2.07 | 0.010916 | WIF1 |
| 17020846 | 8.23 | 9.28 | -2.07 | 0.023421 | COL12A1 |
| 16742286 | 4.35 | 5.42 | -2.11 | 0.034094 | |
| 16777185 | 4.88 | 5.95 | -2.11 | 0.020775 | GJB2 |
| 16973934 | 5.81 | 6.89 | -2.11 | 0.039638 | CYTL1 |
| 17005473 | 6.7 | 7.8 | -2.15 | 0.02775 | SCGN |
| 16674151 | 4.24 | 5.36 | -2.18 | 0.01108 | PAPPA2 |
| 17107855 | 6.76 | 7.92 | -2.23 | 0.02075 | GABRQ |
| 16776103 | 4.76 | 5.94 | -2.26 | 0.034847 | ZIC2 |
| 17055689 | 4.65 | 5.93 | -2.43 | 0.03837 | SP8 |
| 17060039 | 5.13 | 6.45 | -2.5 | 0.011838 | DLX6-AS1 |
| 16703382 | 3.71 | 5.05 | -2.53 | 0.017872 | GAD2 |
| 16837160 | 5.71 | 7.18 | -2.76 | 0.028345 | CACNG5 |
| 16912362 | 4.47 | 5.97 | -2.82 | 0.016906 | ID1 |
| 17118303 | 3.61 | 5.13 | -2.87 | 0.009295 | |

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims. In addition, citation or identification of any reference in this application shall not be construed as an admission that such reference is available as prior art to the present invention.

## Claims

1. An ex vivo method of inducing commitment or maturation of oligodendrocyte cells, the oligodendrocyte cells having been ex-vivo differentiated from glial progenitor cells derived from pluripotent stem cells (PSC) in a medium comprising DMEM/F12, 1% B27, 0.5% N2, Vitamin C, the method comprising: contacting said glial progenitor cells in said medium with LDN-193189.

2. An isolated mature oligodendrocyte cell obtainable according to the method of claim 1, wherein said mature oligodendrocyte cell exhibits a mature oligodendrocyte marker comprising expression of 04, olig2, PDGFRA, O1, PLP, MBP, MAG and MOG.

3. The mature oligodendrocyte cell of claim 2, wherein said mature oligodendrocyte further expresses a marker selected from the group consisting of olig2, olig1, sulfatide marker, galactocerebrosides (O1, GalC), Nkx2.2, Sox10, oligodendrocyte specific protein (OSP), myelin-associated glycoprotein (MAG), 2',3'-cyclic nucleotide-3'-phosphodiesterase (CNP), glutathione-S-transferase (GST), adenomatous polyposis coli (APC); myelin oligodendrocyte glycoprotein (MOG), CNPase, MOSP and Oligodendrocyte NS-1.

4. The mature oligodendrocyte cells of claim 2 for use in treating a medical condition of the CNS in a subject-in-need thereof.

5. A pharmaceutical composition comprising as an active ingredient the committed OPC or mature oligodendrocyte cells of claim 2, and a pharmaceutically acceptable carrier or diluent.

6. The committed or mature oligodendrocyte cell of claim 2, wherein said mature oligodendrocyte cell has a morphology selected from the group consisting of elongated, bipolar or multipolar shape.

7. The committed or mature oligodendrocyte cell of claim 2, wherein said mature oligodendrocyte phenotype comprises in vivo and in vitro myelin production.

8. The committed or mature oligodendrocyte cell of claim 2, wherein said mature oligodendrocyte phenotype comprises a mature oligodendrocyte structural phenotype, preferably wherein said mature oligodendrocyte structural phenotype is branched and ramified.

9. The mature oligodendrocyte cells for use of claim 4, wherein said medical condition is associated with insufficient myelination.

10. The mature oligodendrocyte cells for use of claim 4, wherein said medical condition is selected from the group consisting of multiple sclerosis, stroke, inflammation, spinal cord injury, and an autoimmune disease.

11. A method of determining an effect of a drug on differentiation of human oligodendrocyte progenitor cell toward differentiated and mature oligodendrocyte, the method comprising:
(a) subjecting committed OPC or mature oligodendrocyte according to claim 2 to the drug; and
(b) determining at least one of a structural or functional phenotype of the treated cell as compared to an untreated cell, thereby determining an effect of the drug on the cell functionality.

## Patentansprüche

1. Ex vivo Verfahren zur Induzierung der Weiterentwicklung zu oder Reifung von Oligodendrozyten-Zellen, worin die Oligodendrozyten-Zellen ex-vivo aus von pluripotenten Stammzellen (PSC) abgeleiteten Glia-Vorläufer-Zellen in einem Medium differenziert wurden, umfassend DMEM/F12, 1% B27, 0.5% N2, Vitamin C, wobei das Verfahren umfasst: Inkontaktbringen der Glia-Vorläuferzellen in dem Medium mit LDN-193189.

2. Isolierte reife Oligodendrozyten-Zelle, welche gemäß dem Verfahren nach Anspruch 1 erzeugt wurden, worin die reife Oligodendrozyten-Zelle einen Marker für reife Oligodendrozyten aufweist, welche die Expression von 04, olig2, PDGFRA, O1, PLP, MBP, MAG und MOG umfasst.

3. Reife Oligodendrozyten-Zelle nach Anspruch 2, worin der reife Oligodendrozyt weiter einen Marker exprimiert ausgewählt aus der Gruppe bestehend aus olig2, olig1, Sulfatid-Marker, Galactocerebrosid (O1, GalC), Nkx2.2, Sox10, Oligodendrozytenspezifisches Protein (OSP), Myelin-assoziiertes Glycoprotein (MAG), 2',3'-zyklische Nucleotid-3'-phosphodiesterase (CNP), Glutathion-S-transferase (GST), adenomatöse Polyposis coli (APC); Myelin-Oligodendrozyten-Glycoprotein (MOG), CNPase, MOSP und Oligodendrozyt NS-1.

4. Reife Oligodendrozyten-Zelle nach Anspruch 2 zur Verwendung bei der Behandlung eines medizinischen Zustandes des ZNS in einem dies benötigenden Individuum.

5. Pharmazeutische Zusammensetzung, welche als aktiven Inhaltsstoff die weiterentwickelte OPC oder Oligodendrozyten-Zelle nach Anspruch 2 enthält, und einen pharmazeutisch annehmbaren Träger oder Verdünnungsmittel.

6. Weiterentwickelte oder reife Oligodendrozyten-Zelle nach Anspruch 2, worin die reife Oligodendrozyten-Zelle eine Morphologie aufweist, ausgewählt aus der Gruppe bestehend aus einer länglichen, bipolaren oder multipolaren Form.

7. Weiterentwickelte oder reife Oligodendrozyten-Zelle nach Anspruch 2, worin der reife Oligodendrozyt-Phänotyp eine Myelin-Produktion in vivo und in vitro umfasst.

8. Weiterentwickelte oder reife Oligodendrozyten-Zelle nach Anspruch 2, worin die reife Oligodendrozyten-Zelle einen strukturellen Phänotyp eines reifen Oligodendrozyten umfasst, vorzugsweise worin der strukturelle Phänotyp des reifen Oligodendrozyten verzweigt und verästelt ist.

9. Reife Oligodendrozyten-Zellen zur Verwendung nach Anspruch 4, worin der medizinische Zustand mit unzureichender Myelinisierung assoziiert ist.

10. Reife Oligodendrozyten-Zellen zur Verwendung nach Anspruch 4, worin der medizinische Zustand ausgewählt ist aus der Gruppe bestehend aus multipler Sklerose, Schlaganfall, Entzündung, Verletzung des Rückenmarks, und einer Autoimmunerkrankung.

11. Verfahren zur Bestimmung der Auswirkung eines Arzneimittels auf die Differenzierung einer humanen Oligodendrozyten-Vorläuferzelle zu einem differenzierten und reifen Oligodendrozyten, wobei das Verfahren umfasst:
(a) Unterwerfen einer weiterentwickelten OPC oder reifen Oligodendrozyten-Zelle nach Anspruch 2 dem Arzneimittel; und
(b) Bestimmen mindestens eines strukturellen oder funktionellen Phänotyps der behandelten Zelle im Vergleich mit einer unbehandelten Zelle, wodurch eine Auswirkung des Arzneimittels auf die Zellfunktionalität bestimmt wird.

## Revendications

1. Procédé ex vivo d'induction de détermination ou de maturation de cellules d'oligodendrocyte, les cellules d'oligodendrocyte ayant été différenciées ex vivo à partir de cellules progénitrices gliales issues de cellules souches pluripotentes (CSP) dans un milieu comprenant du DMEM/F12, 1% de B27, 0,5% de N2, de la vitamine C, le procédé comprenant : la mise en contact desdites cellules progénitrices gliales dans ledit milieu avec du LDN-193189.

2. Cellule d'oligodendrocyte mature isolée pouvant être obtenue selon le procédé de la revendication 1, ladite cellule d'oligodendrocyte mature présentant un marqueur d'oligodendrocyte mature comprenant l'expression d'O4, d'olig2, de PDGFRA, d'O1, de PLP, de MBP, de MAG et de MOG.

3. Cellule d'oligodendrocyte mature selon la revendication 2, ledit oligodendrocyte mature exprimant en outre un marqueur choisi dans le groupe constitué par olig2, olig1, marqueur sulfatide, galactocérébrosides (O1, GalC), Nkx2.2, Sox10, protéine spécifique d'oligodendrocyte (PSO), glycoprotéine associée à la myéline (MAG), 2'3'-nucléotide cyclique-3'-phosphodiestérase (CNP), glutathione-S-transférase (GST), polypose adénomateuse (PA) ; glycoprotéine d'oligodendrocyte de myéline (MOG), CNPase, MOSP et oligodendrocyte NS-1.

4. Cellules d'oligodendrocyte matures selon la revendication 2 pour une utilisation dans le traitement d'une affection médicale du SNC chez un sujet en ayant besoin.

5. Composition pharmaceutique comprenant en tant que principe actif les CPO déterminées ou les cellules d'oligodendrocyte matures selon la revendication 2 et un véhicule ou un diluant pharmaceutiquement acceptable.

6. Cellule d'oligodendrocyte déterminée ou mature selon la revendication 2, ladite cellule d'oligodendrocyte mature présentant une morphologie choisie dans le groupe constitué par une forme allongée, une forme bipolaire ou une forme multipolaire.

7. Cellule d'oligodendrocyte déterminée ou mature selon la revendication 2, ledit phénotype d'oligodendrocyte mature comprenant la production in vivo et in vitro de myéline.

8. Cellule d'oligodendrocyte déterminée ou mature selon la revendication 2, ledit phénotype d'oligodendrocyte mature comprenant un phénotype structural d'oligodendrocyte mature, préférablement, ledit phénotype structural d'oligodendrocyte mature étant divisé et ramifié.

9. Cellules d'oligodendrocyte matures pour une utilisation dans la revendication 4, ladite affection médicale étant associée à une myélinisation insuffisante.

10. Cellules d'oligodendrocyte matures pour une utilisation dans la revendication 4, ladite affection médicale étant choisie dans le groupe constitué par la sclérose en plaques, un accident vasculaire cérébral, une inflammation, une lésion de la moelle épinière et une maladie auto-immune.

11. Procédé de détermination d'un effet d'un médicament sur la différenciation de cellules progénitrices d'oligodendrocyte humaines en oligodendrocyte différencié et mature, le procédé comprenant :
(a) la soumission des CPO déterminées ou de l'oligodendrocyte mature selon la revendication 2 au médicament ; et
(b) la détermination d'au moins l'un parmi un phénotype structural ou fonctionnel de la cellule traitée par rapport à une cellule non traitée, ce qui permet de déterminer un effet du médicament sur la fonctionnalité cellulaire.
